Europäisches Patentamt

European Patent Office

Office européen des brevets

(19)

(11) Publication number: **0 052 001**
**B1**

(12) **EUROPEAN PATENT SPECIFICATION**

(45) Date of publication of patent specification: 01.08.84

(51) Int. Cl.³: **C 12 P 1/00**, C 12 P 19/44,
C 12 N 5/00, C 12 N 11/00
//C12R1/00

(21) Application number: 81305301.4

(22) Date of filing: 06.11.81

(54) Callus culture in nutrient flow.

(30) Priority: 06.11.80 GB 8035626

(43) Date of publication of application:
19.05.82 Bulletin 82/20

(45) Publication of the grant of the patent:
01.08.84 Bulletin 84/31

(84) Designated Contracting States:
AT BE CH DE FR GB IT LI LU NL SE

(56) References cited:
DE - A - 2 658 894

CHEMICAL ABSTRACTS, vol. 91, no. 11, 10th
September 1979, page 440, no. 87596z
Columbus, Ohio, U.S.A. P. BRODELIUS et al.:
"Immobilized plant cells for the production and
transformation of natural products"
CHEMICAL ABSTRACTS, vol. 63, 1965, column
19001b-e Columbus, Ohio, U.S.A.
BIOTECHNOLOGY AND BIOENGINEERING, vol.
19, 1977, pages 387-397 M. KIERSTAN et al.:
"The immobilization of microbial cells,
subcellular organelles, and enzymes in calcium
alginate Gels"

(73) Proprietor: Albright & Wilson Limited
Albright & Wilson House Hagley Road West
Oldbury Warley West Midlands, B68 ONN (GB)

(72) Inventor: Childs, Anthony Francis
The Meads, Beech Close Kinver
Stourbridge West Midlands, DY7 6LW (GB)
Inventor: Yeoman, Michael Magson
9 Glenlockhart Valley
Edinburgh, EH 141 DE (GB)
Inventor: Fagandini, Danilo A.A.
6 Alleyn Park Dulwich
London SE21 (GB)

(74) Representative: Savidge, Roger Gordon Madgwick
et al,
c/o Albright & Wilson Limited 1 Knightsbridge
Green
London SW1X 7QD (GB)

(56) References cited:
BIOTECHNOLOGY AND BIOENGINEERING
SYMPOSIUM, no. 3, 1972, Interscience
Publishers M.C. PORTER: "Applications of
membranes to enzyme isolation and
purification"

Courier Press, Leamington Spa, England.

## Description

This invention provides a method and apparatus for growing plant tissue cultures and for using them in the production of secondary metabolites. It is known that plant cells isolated from the parent plant can be induced to grow and multiply in or on a suitable medium to form a sustainable growth of plant callus tissue. A callus is an aggregate of substantially undifferentiated, unspecialised plant cells ("callus cells") which are capable, depending upon the physical or chemical environment in which they are placed, of either multiplying or of developing into any of the more specialised plant cells, and, ultimately into a complete plant.

Three methods of growing plant tissue cultures are commonly practised. The cells may be grown in suspension in an aqueous nutrient solution, or on a bed of a nutrient substrate such as agar jelly or encapsulated in beads of polymer. The first method has the advantage of permitting close control over the cell's environment and is particularly suitable for maintaining the cells in an undifferentiated and rapidly multiplying state. The second method has the disadvantage that the environment of the growing cells cannot be closely controlled, and toxic metabolic products accumulate in the substrate. Cultures grown under the latter conditions from coherent pieces of callus tissue, which in time begin to exhibit signs of differentiation and a slowing of growth. The third method inhibits direct contact between the cells and the medium and the interaction of the cells to form compact callus tissue.

The primary products of plant metabolites are proteins and carbohydrates. However, there are many important plant products which fall into neither of those categories, and they are conveniently referred to as secondary metabolites.

For many years attempts have been made to recover secondary metabolites from plant cell cultures. Plant secondary metabolites include a very wide range of galenicals, essential oils and resins which are of great commercial importance in the pharmaceutical, perfumery and flavouring industries. However, attempts to extract such metabolites from suspensions of plant cells have hitherto been unsuccessful, except in the case of one or two types of plant. It has appeared that most plants do not provide cell cultures capable of forming useful secondary metabolites, at least under the conditions of culture that have been employed. We have now discovered that when plant tissue cultures are grown as calli supported on the surface of a substrate and in direct contact with a nutrient medium, the production of secondary metabolites is enhanced compared with cultures grown in suspension or encapsulated. We have further discovered that plant tissue cultures may be efficiently grown under controlled nutrient conditions and with enhanced yields of secondary metabolites when callus tissue is maintained substantially stationary with respect to the substrate and is contacted with a circulating aqueous nutrient solution.

Generally, our invention provides a method for the production of plant metabolites which comprises growing calli on the surface of an inert substrate with respect to which the callus tissue is substantially stationary, contacting the calli directly with a flow of aerated nutrient solution and recovering metabolites from the culture and/or the solution.

According to one particular aspect, our invention provides a method for the growth of plant tissue cultures which comprises supporting a growing culture of plant callus cells on a substantially horizontal bed, continuously or intermittently supplying to the bed an aqueous solution of nutrients for the culture, passing the solution over or through the bed, contacting the culture with the solution and with air and continuously or intermittently removing the solution from the bed.

According to a second particular embodiment, the invention provides apparatus for the growth of plant tissues cultures which comprises a sterilisable system including a closed vessel, a water-permeable bed in the vessel adapted to support growing callus cultures, an inlet for supplying nutrient solution to the bed, and means for withdrawing the solution from the bed.

Alternatively, and preferably for commercial production, a column may be packed with a particulate or fibrous substrate, and the callus tissue grown thereon. Aerated nutrient solution may be passed through the column, e.g. nutrient solution may be passed down the column and air bubbled up through the column.

For example a column packed with alginate supported on an inert fibrous base such as polyamide fibre has been found to provide an effective substrate.

This particulate substrate may alternatively be porous and/or hollow to permit callus growth within the substrate and at the same time to permit contact between the callus and the aerated nutrient solution. Preferably the substrate may comprise bundles of inert (e.g. polyamide) fibre or wire mesh, such as may be used as pan scrubbers, preferably rigid, or hollow perforated spheres such as those used as balls for practicing golf, or a polymer foam with an open cellular structure. Such porous or hollow supports may be invasible by the callus tissue.

It is particularly preferred to maintain a stirred, air agitated or liquid fluidised system in which callus tissue supported on a particulate substrate as aforesaid is maintained in suspension in the nutrient fluid, in a reactor or tank through which nutrient may circulate.

Preferably the system includes means for circulating the nutrient solution from a reservoir to the bed or column and for returning the solution withdrawn therefrom to the reservoir. Preferably means are provided for introducing fresh nutrients to the system or for withdrawing part of the solution, for dumping, cleaning or recovery of secondary metabolites. Circulation of the nutrient solution may conveniently be affected by a peristaltic pump.

It is possible to pump liquor both to and from the bed reactor or column, for example using two channels of a multi-channel pump. Preferably, however, flow in one direction is by gravity, maintaining the substrate and upper level of liquid in the reservoir at different levels and establishing a pumped flow from the lower of the two to the higher and a gravity flow in the reverse direction via an overflow or constant head outlet. Most conveniently the reservoir is at a higher lever than the substrate and liquid flows from the reservoir through a substantially upright or upwardly slanting outlet tube, the upper end of which is situated within the reservoir, and the height of which can be adjusted to control the volume of liquid in the reservoir, and hence the proportion of liquid in contact with the substrate.

The substrate is desirably constructed of a water-permeable and, preferably, biologically inert material. Preferably the substrate is of a material that will withstand autoclaving, for easy sterilisation. Where a fixed bed is employed, it may, for example, be of a particulate material such as sand or alumina, or a porous material such as sintered glass. Alternatively, or where a system is employed in which particles of substrates are in motion relative to each other, the substrate may comprise a fibrous material such as glass fibre, cellulose or synthetic organic fibres, or a perforated hollow plastic sphere.

The callus may be grown on a bed which is sufficiently porous to permit invasion of the bed by the callus tissue. Otherwise the bed is preferably substantially horizontal, since the growing callus may have relatively little capacity to adhere to the bed, and cannot easily be supported without slipping or rolling if the bed slopes at too steep an angle (e.g. more than about 30°C). However, there is sometimes an advantage inclining the bed at a very small angle e.g. 2 to 10°, to the horizontal to provide drainage and prevent flooding of the bed due to any inequalities of flow.

The nutrient solution may be dipped or sprayed onto the bed through a feed inlet and removed, e.g. by suction through a tube immersed in the bed or by overflowing through an outlet at the level of the bed. Alternatively the bed may be supported on or may comprise a perforated or sintered plate through which the nutrient solution may drain into an outlet duct. In the latter case the bed may be irrigated from below e.g. by a reciprocating flow of the nutrient solution through a duct below the bed, alternately flooding and draining it.

While there is no theoretical limit to the size of the bed it will generally be convenient for large scale operation on a fixed bed to use relatively small units, arranged in parallel and/or in series, both for ease of sterilisation and to help localise and isolate any infection. For example, a number of beds may be supplied from a single reservoir via an inlet manifold and the fluid in the bed may drain, overflow or be pumped into a outlet manifold. Alternatively, it is possible to arrange a number of beds in cascade, one above the other, allowing the nutrient solution to trickle down the system by gravity. For example, the beds may be slightly tilted in alternate directions down the cascade so that liquid drains from the lower edge of each bed onto the upper edge of the next lower bed, or may comprise perforated plates through each of which the liquid drips onto the bed below.

The rate of flow of the nutrient solution through a fixed bed system should not be so rapid as to disturb or dislodge the culture, but should be sufficient to maintain a substantially constant supply of nutrients to the callus, and to prevent any harmful build up of metabolites. The depth of the nutrient solution in the bed is preferably sufficient to contact and wet the callus tissue without totally immersing it, in order to permit sufficient contact between the cells and the air.

According to an alternative embodiment, the callus tissue may be supported or suspended on or in a particulate or granular, hollow or porous substrate in a moving mixture of air and nutrient solution. Such a system has the advantage of permitting undisturbed growth of the callus tissue within the substrate, while the relative motion of the particles prevents excessive growth, abrading any outgrowth of tissue so preserving the permeability of the system to air and nutrient flows.

An important advantage of the present method is that it permits controlled variations of the nutrient regime supplied to the culture. This has been found valuable in inducing the production of secondary metabolites by cultures which do not normally yield them when grown under the normal conditions of suspension culture.

Typically, cells are first grown by conventional suspension culture techniques to promote optimum reproduction of a healthy cell population. The substrate is then innoculated with the cells and culture in accordance with the invention is commenced.

Preferably the culture is initially fed a solution adapted to promote optimum growth of the unspecialised cells, until a healthy growth has been established, and the composition of the nutrient solution is then gradually altered to optimise production of the desired secondary metabolite. For example, precursors of the desired metabolite or development modifiers such as auxins, cytokinins and gibberellins may be added to the solution, and/or the concentrations of selected nutrients or development modifiers in the solution may be reduced to inhibit competing biochemical pathways.

The supply of air may be controlled in various ways. In general callus culture cannot be conducted in totally anaerobic conditions. Often sufficient aeration is achieved by permitting some contact between the callus and the atmosphere, or by bubbling air through the nutrient solution or even allowing the nutrient to absorb some air from the atmosphere prior to contacting the calli. Production of secondary metabolites may be favoured by restricting the air supply to the culture.

Illumination can profoundly affect the development of plant tissue cultures. It is sometimes preferred that the vessel containing the culture should be transparent to permit illumination of the growing cells. Normally cultures will be grown in artificially simulated white light, but illumination by selected wavelengths or growth in darkness or under cycles of illumination may in some instances be preferred in order to favour production of specific metabolites. Preferably means are provided to control the temperature of the culture, which also affects the rate of growth and the yield of secondary metabolites.

We have found that our invention is useful for the production of a number of plant secondary metabolites. For example, cells of certain Capsicum species e.g. *Capsicum frutescens* or *Capsicum annuum* can be grown according to our invention and induced to form significant yields of capsaicinoids, the principle active compounds in the flavour of chilli pepper. The same cells do not yield capsaicinoids in suspension cultures. According to a further embodiment therefore our invention provides a method for the production of capsaicinoids which comprises forming a culture of callus tissue from a capsaicinoids producing species of *Capsicum*, supporting the culture on a substrate in contact with air and with an aqueous nutrient solution and removing capsaicinoids from the culture. The term "Capsaicinoids" is used herein generically to include the various components of commercial capsaicin including the compound capsaicin itself, dihydrocapsaicin, nor-dihydrocapsaicin homo-capsaicin, homo-dihydrocapsaicin.

Preferably, to stimulate production of capsaicinoids, precursors such as valine and phenylalanine are added to the nutrient solution, once a healthy growth has been established, and competing protein synthesis is checked by progressively reducing the nitrate and/or carbohydrate content of the solution. This may be achieved by allowing the culture to deplete the solution naturally, and then adding the precursor. We have found the particularly high yields of capsaicin are obtained when isocapric acid is added as precursor to the nutrient medium. Water soluble salts such as sodium or potassium isocaprate may also be used.

The invention may also be used to stimulate the production of diosgenin by tissue cultures obtained from diosgenin-forming strain of yam of the genus Dioscorea. Diosgenin is widely used industrially for the synthesis of certain commercially valuable steroids.

Other secondary metabolites which may be prepared in accordance with the invention include L-DOPA, the constituents of rose oil, various alkaloids such as opium alkaloids and derivatives of datura.

Secondary metabolites may be recovered from cultures grown according to our invention, either from the circulating solution (e.g. by filtering off suspended dead cells, or by evaporation, selective absorption, precipitation, dialysis, reverse osmosis, solvent extraction or any other convenient separatory method) or by harvesting the cultures and extracting the desired products, depending upon the extent to which they are excreted. In many cases the metabolites may be removed from the callus by plasmolysis, which involves contacting the cell with an aqueous solution of a non-toxic, nonionic compound at a concentration sufficient to promote osmotic shrinkage of the cell, which may be accompanied by excretion of secondary metabolites.

The invention is particularly useful for systems in which the desired metabolite is excreted into the nutrient medium, and we prefer in such instances to recover the metabolite continually or intermittently from the nutrient medium while it is recycling. This reduces the inhibition of the desired metabolic pathways by feedback mechanisms caused by excessive build up of the metabolite in the nutrient.

Suitable nutrient solution for the growth of callus cultures are well known in the art. A typical example is described by Marashige and Skoog in Physicologa Plantarum Vol. 15 pp 473—497. Such solutions may be supplemented by additions of natural plant fluids such as coconut milk, or by yeast or malt extracts.

Growth of plant tissue cultures should, of course, be conducted in sterile conditions to avoid contamination of the culture and its nutrient medium.

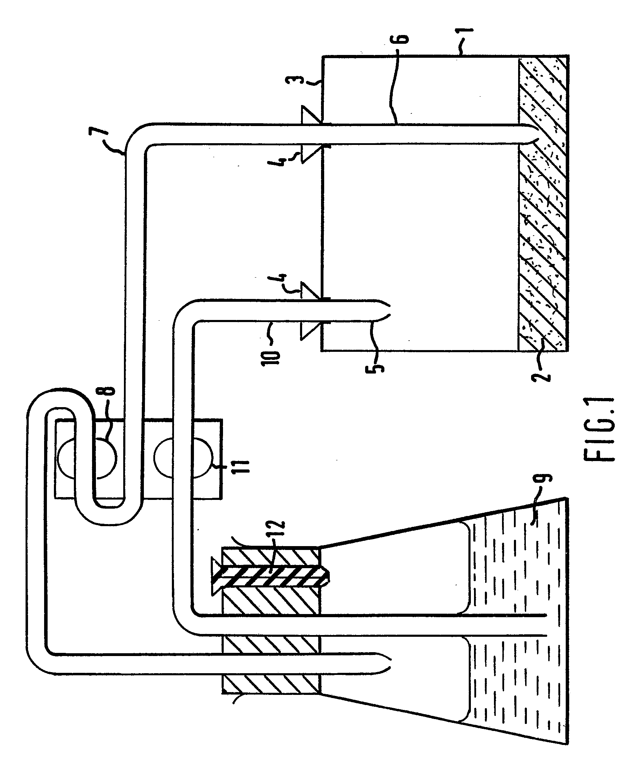

One form of apparatus according to the invention will be described with reference to Figure 1 of the accompanying drawings which is a diagrammatic cross section. The apparatus comprises a glass vessel (1) containing a bed (2) of fibres of the type sold commercially by Borden under the trade mark "Safe Grow" water mat. The vessel (1) is enclosed by a lid (3), provided with two rubber gaskets (4) through which pass a steel inlet tube (5) and a steel outlet tube (6) which extends into the bed. A recycle line (7) connects the outlet tube (6), via a peristaltic pump (8), with a reservoir (9). A feed line (10) connects the reservoir (9) via a peristaltic pump (11) with the inlet 5. The reservoir 9 is provided with a self sealing rubber gasket (12), through which the contents of the reservoir may be withdrawn or fresh nutrient introduced by means of a hypodermic syringe.

The pumps 8 and 11 are conveniently separate channels of a multi-channel peristaltic pump.

In use, after the apparatus has been thoroughly sterilised, the reservoir 9 is charged with nutrient solution which is pumped by the pump 11 through the feed line 10 to the inlet 5. The solution drips onto the bed 2 until it is saturated. Surplus solution sucked out of the bed through the outlet tube 6 and pumped by the pump 8 through the recycle line 7 to the reservoir 9.

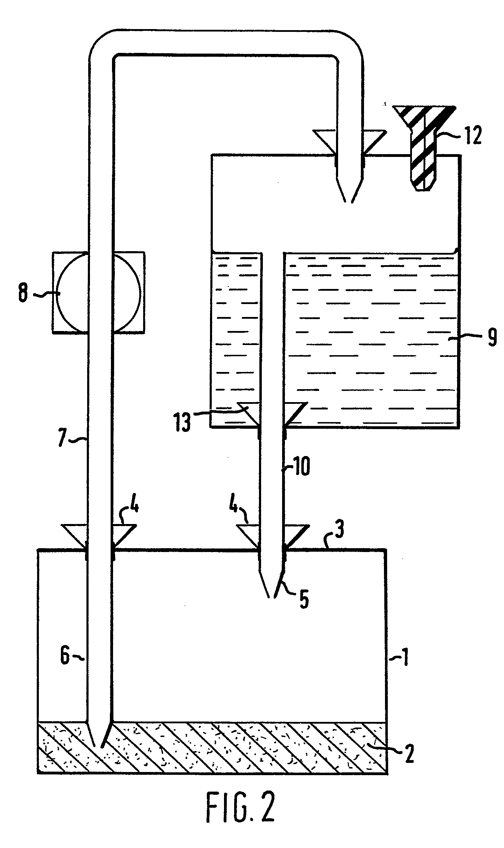

An alternative embodiment is illustrated in Figure 2 of the drawings which is also a diagrammatic cross section. This embodiment is substantially similar to that of Figure 1, with the elements similarly numbered, except that the pump is omitted, the reservoir being situated above the bed, and the feed line (10) is a substantially vertical tube whose upper end projects into the reservoir 9 through a gasket (13) in the base thereof, and whose lower end projects through the lid 3 of the vessel 1 and constitutes the inlet tube 5.

In use, solution from the reservoir overflows into the bed through the tube 10 and is continuously recycled through the line 7 by the pump 8.

The height of the tube 10 can be adjusted thereby raising or lowering the maximum level of liquid in the reservoir. This in turn determines what proportion of the circulating fluid will be present in the vessel 1.

The invention will be illustrated by the following examples:—

Using the apparatus hereinbefore described with reference to Figure 1, calluses of *Capsicum annuum* were cultured (Example 1) at 25°C under an artificial illumination of 500 lux and in Marashige and Skoog nutrient solution, without added growth factors. For comparison a second culture (Example II) was grown under the same conditions but with no nitrogen in the nutrient medium. Radioactively labelled phenylalanine and valine were added to the nutrient medium. The medium and the culture were analysed by thin layer chromatography on a silica gel plate with silver nitrate and boric acid. The samples were eluted firstly with a solution consisting of equal proportions by weight of chloroform and ethyl acetate and secondly, at right angles, with a solvent consisting of 95 parts by weight chloroform, 5 parts methanol and 1 part glacial acetic acid. Spots due to capsaicin, dihydrocapsaicin and cinnamic acid were identified. The capsaicin and dihydrocapsaicin counts were totalled and recorded in the following table:—

| | Total counts added to nutrient | Capsaicin counts in callus | Capsaicin counts in medium | Residual counts in callus |
|---|---|---|---|---|
| Example I | $5 \times 10^6$ | $4.5 \times 10^2$ | $418 \times 10^3$ | $28 \times 10^4$ |
| Example II | $5 \times 10^6$ | $22.3 \times 10^3$ | $499 \times 10^3$ | $820$ |

By comparison no capsaicin production was observed in a suspension culture under similar conditions. A static callus grown on agar jelly formed very low levels of capsaicin. The examples illustrated the effectiveness of calli grown according to the invention in forming secondary metabolite and the increased efficiency with which the radioactively labelled precursors (valine and phenylalanine) are converted to the desired secondary metabolite (capsaicin) when protein formation is halted by removal of nitrogen from the nutrient.

Example III

A glass column 20 cm by 2.5 cm was packed with pan scrubbers each consisting of a toroidal bundle of coarse polyamide fibre. The pan scrubbers were first inoculated by immersion in a suspension of *capsicum annuum* cells in sodium alginate solution. The pan scrubbers were then contacted with aqueous calcium chloride solution thereby precipitating calcium alginate on the fibres.

50 mls of Marashige and Skoog nutrient solution were circulated through the column for 5 days under constant artificial illumination at 25°C. During this period air was blown through the column.

5 mm of iso-capric acid was added to the nutrient and circulation resumed for two weeks, without air supply to the column other than that absorbed by the nutrient on contact with the atmosphere.

The nutrient solution was extracted with chloroform to yield 350 micrograms of capsaicinoids.

**Claims**

1. A method for the production of plant metabolites which comprises growing calli on the surface of a inert substrate with respect to which the callus tissue is substantially stationary, contacting the calli directly with a flow of aerated nutrient solution and recovering metabolites from the culture and/or the solution.

2. A method according to claim 1, wherein callus tissue is supported on a substantially horizontal bed, the bed is continuously or intermittently supplied with an aqueous solution of nutrients for the callus culture, the solution is passed over or through the bed and the callus culture is supplied with the solution and with air and the solution is continuously or intermittently removed from the bed.

3. A method according to claim 1, wherein callus tissue is grown in a column packed with a particulate or fibrous substrate, aerated nutrient solution is passed through the column and metabolites are extracted from the aqueous solution, and/or tissue.

4. A method according to either of claims 1 and 3, wherein callus tissue is grown in a porous or hollow particulate substrate.

5. A method according to claim 4, wherein the substrate is sufficiently porous to permit invasion thereof by plant cells, and the growth of calli therein.

6. A method according to claim 4 or 5, wherein the substrate comprises bundles of an inert fibre or wire.

7. A method according to claim 1, 4 or 5, wherein the substrate comprises hollow perforated spheres.

8. A method according to claim 1, 4 or 5, wherein the substrate comprises particles or a polymer foam having an open cellular structure.

9. A method according to any of claims 5 to 8, wherein the bed of particulate substrate comprises a stirred air agitated or liquid fluidised system in which the callus tissue supported on the particulate substrate is maintained in suspension in the circulating nutrient fluid.

10. A method according to any foregoing claim, wherein plant cells are grown in suspension culture and the callus culture is established by inoculating said substrate with cells from said suspension culture.

11. A method according to any foregoing claim, wherein the callus culture is grown initially in a medium adapted to promote rapid growth thereof and subsequently in a medium containing a precursor for said metabolite.

12. A method according to claim 11, wherein during the latter stages of growth of the callus culture the nutrient medium is depleted with respect to ingredients required for sustained growth.

13. A method according to claim 12, where said nutrient medium is depleted with respect to nitrates and/or carbohydrates.

14. A method according to any foregoing claim, wherein the plant callus culture comprises calli of a capsaicinoids producing strain of ths genus *capsicum* and wherein said metabolite comprises capsaicinoids.

15. A method according to claim 14, wherein the nutrient solution comprises, at least in the latter stages of the culture, iso-capric acid or a water soluble salt thereof.

## Patentansprüche

1. Verfahren zur Herstellung von Stoffwechselprodukten von Pflanzen, dadurch gekennzeichnet, daß man Kalli der Pflanze auf der Oberfläche eines inerten Substrats wachsen läßt, gegenüber welchem sich das Kallusgewebe in wesentlichem im stationären Zustand befindet, daß man die Kalli unmittelbar mit einem Strom einer belüfteten Nährlösung in Kontakt bringt und die Stoffwechselprodukte aus der Kultur und/oder aus der Lösung isoliert.

2. Verfahren nach Anspruch 1, dadurch gekennzeichnet, daß man das Kallusgewebe auf einem im wesentlichen horizontalen Bett fixiert und das Bett kontinuierlich oder absatzweise mit einer wäßrigen Lösung von Nährstoffen für die Kalluskultur beschickt, die Lösung über das Bett oder durch das Bett hindurch leitet und die Kalluskultur mit der Lösung und mit Luft beschickt und die Lösung kontinuierlich oder absatzweise vom Bett abzieht.

3. Verfahren nach Anspruch 1, dadurch gekennzeichnet, daß man das Kallusgewebe in einer Säule wachsen läßt, die mit einem teilchenförmigen oder faserförmigen Substrat gepackt ist, daß man belüftete Nährlösung durch die Säule laufen läßt und die Stoffwechselprodukte aus der wäßrigen Lösung und/oder dem Gewebe extrahiert.

4. Verfahren nach Anspruch 1 oder Anspruch 3, dadurch gekennzeichnet, daß man das Kallusgewebe in einem porösen oder hohlen teilchenförmigen Substrat wachsen läßt.

5. Verfahren nach Anspruch 4, dadurch gekennzeichnet, daß das Substrat ausreichend porös ist, um das Einwandern von Pflanzenzellen in die Poren und das Wachsen von Kalli darin zu gestatten.

6. Verfahren nach Anspruch 4 oder 5, dadurch gekennzeichnet, daß das Substrat aus Bündeln einer inerten Faser oder eines inerten Drahts besteht.

7. Verfahren nach den Ansprüchen 1, 4 oder 5, dadurch gekennzeichnet, daß das Substrat aus hohlen, perforierten Kugeln besteht.

8. Verfahren nach den Ansprüchen 1, 4 oder 5, dadurch gekennzeichnet, daß das Substrat aus Teilchen eines Polymerschaumstoffs mit offenzelliger Struktur besteht.

9. Verfahren nach den Ansprüchen 5 bis 8, dadurch gekennzeichnet, daß das Bett aus teilchenförmigem Substrat aus einem gerührten, mittels Luft bewegten oder flüssigen fluidisierten System besteht, in welchem das Kallusgewebe, das vom teilchenförmigen Substrat gestützt ist, in Suspension in der umlaufenden Nährflüssigkeit gehalten wird.

10. Verfahren nach den vorhergehenden Ansprüchen, dadurch gekennzeichnet, daß Pflanzenzellen in der Suspensionskultur wachsen gelassen werden und die Kalluskultur durch Impfen des Substrats mit Zellen aus der Suspensionskultur angelegt wird.

11. Verfahren nach den vorhergehenden Ansprüchen, dadurch gekennzeichnet, daß die Kalluskultur anfangs in einem Medium wachsen gelassen wird, das das schnelle Wachstum der Kultur fördert, und anschließend in einem Medium wachsen gelassen wird, das einen Vorläufer des Stoffwechselprodukts enthält.

12. Verfahren nach Anspruch 11, dadurch gekennzeichnet, daß während der letzten Wachstumsstufen der Kalluskultur das Medium bezüglich der für ein gebremstes Wachstum benötigten Bestandteile verarmen gelassen wird.

13. Verfahren nach Anspruch 12, dadurch gekennzeichnet, daß das Nährmedium bezüglich Nitraten und/oder Kohlehydraten verarmen gelassen wird.

14. Verfahren nach den vorhergehenden Ansprüchen, dadurch gekennzeichnet, daß die Pflanzenkalluskultur aus Kalli eines Capsaicine erzeugenden Stammes der Pflanzengattung Capsicum besteht und die genannten Stoffwechselprodukte Capsaicine sind.

15. Verfahren nach Anspruch 14, dadurch gekennzeichnet, daß die Nährlösung wenigstens in den letzten Stufen der Kultur Isocaprinsäure oder ein wasserlösliches Salz davon enthält.

## Revendications

1. Procédé pour la production de métabolites de plantes caractérisé en ce que l'on fait croître des calus à la surface d'un substrat inerte par rapport auquel le tissu de calus est sensiblement immobile, met les calus directement en contact avec un courant de solution nutritive aérée et récupére les métabolites à partir de la culture et/ou de la solution.

2. Procédé selon la revendication 1, caractérisé en ce que le tissu de calus est maintenu sur un lit sensiblement horizontal, que le lit est alimenté en continu ou par intermittence par un solution aqueuse de substances nutritives pour la culture de calus, que l'on fait passer la solution au-dessus ou à travers le lit, que la culture de calus est alimentée par la solution et de l'air et que la solution est retirée du lit en continu ou par intermittence.

3. Procédé selon la revendication 1, caractérisé en ce que du tissu de calus est cultivé dans une colonne garnie d'un substrat fibreux ou en particules, qu'une solution nutritive aérée est envoyée dans la colonne et que les métabolites sont extraits de la solution aqueuse et/ou du tissu.

4. Procédé selon l'une quelconque des revendications 1 à 3, caractérisé en ce que du tissu de calus est cultivé dans un substrat en particules poreux ou creux.

5. Procédé selon la revendication 4, caractérisé en ce que le substrat est suffisamment poreux pour permettre son envahissement par des cellules végétales et la croissance de calus à l'intérieur du substrat.

6. Procédé selon la revendication 4 ou 5, caractérisé en ce que le substrat est constitué par des faisceaux de fibres inertes ou par un treillis metallique.

7. Procédé selon la revendication 1, 4 ou 5, caractérisé en ce que le substrat est constitué par des sphères creuses perforées.

8. Procédé selon la revendication 1, 4 ou 5, caractérisé en ce que le substrat est constitué par des particules d'une mousse de polymère possédant une structure à cellules ouvertes.

9. Procédé selon l'une quelconque des revendications 5 à 8, caractérisé en ce que le lit de substrat en particules comprend un système liquide fluidisé, brassé ou agité par de l'air dans lequel le tissu de calus déposé sur le substrat en particules est maintenu en suspension dans le fluide nutritif en circulàtion.

10. Procédé selon l'une quelconque des revendications précédentes, caractérisé en ce que l'on fait croître des cellules végétales par culture en suspension et que l'on produit la culture de calus en inoculant audit substrat des cellules provenant de ladite culture en suspension.

11. Procédé selon l'une quelconque des revendications précédentes, caractérisé en ce que l'on fait croître au départ la culture de calus dans un milieu apte à favoriser sa croissance rapide et ensuite dans un milieu contenant un précurseur pour ledit métabolite.

12. Procédé selon la revendication 11, caractérisé en ce qu'au cours des derniers stades de croissance de la culture de calus, le milieu nutritif est appauvri en ce qui concerne les constituants nécessaires à une croissance entretenue.

13. Procédé selon la revendication 12, caractérisé en ce que le milieu nutritif est appauvri en nitrates et/ou en hydrates de carbone.

14. Procédé selon l'une quelconque des revendications précédentes, caractérisé en ce que la culture de calus de plantes comprend des calus d'une souche génératrice de capsaïcinoïdes de l'espèce *capsicum* et que ledit métabolite comprend des capsaïcinoïdes.

15. Procédé selon la revendication 14, caractérisé en ce que la solution nutritive contient, du moins dans les derniers stades de la culture, de l'acide isocaprique ou un de ses sels solubles dans l'eau.

FIG.1

0 052 001

FIG. 2